# EUROPEAN PATENT APPLICATION

(11) **EP 1 411 041 A1**
(43) Date of publication of application: **21.04.2004**
(21) Application number: 02292585.3
(22) Date of filing: 18.10.2002
(51) Int. Cl.: C07C 59/42, G01N 33/50

(54) **Specific markers of lipid peroxidation**

(71) Applicant: INSTITUT NATIONAL DE LA SANTE ET DE LA RECHERCHE MEDICALE (INSERM), 75654 Paris Cédex 13 (FR); Institut National des Sciences Appliquées de Lyon, 69621 Villeurbanne (FR)
(72) Inventor: Lagarde Michel, 69150 Decines-Charpieu (FR); Deshayes Christian, 69100 Villeurbanne (FR); Guichardant Michel, 69370 Saint-Didier-au-mont-d'or (FR); Chantegrel Bernard, 69100 Villeurbanne (FR)
(74) Representative: Breesé, Pierre

(57) **Abstract**

The present invention concerns a 4-hydroxy-2E-alkenoic acid according to formula (I) below 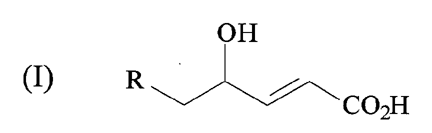 in which R is chosen in the group consisting of CH₃-, CH₃-(CH₂)₃- and CH₃-(CH₂)₃-CH=CH- and its use as lipid peroxidation marker. The present invention also concerns methods to determine its presence and its level in solution and methods for the diagnosis of a pathology involving an increase in lipid peroxidation and for following the effectiveness of the treatment of said pathologies.

## Description

The present invention concerns new specific markers of lipid peroxidation. These markers are 4-HHA, 4.-HNA and the 4-HDDA. The present invention also concerns methods to determine their presence and their level in solution. The present invention provides methods for the diagnosis of pathologies involving an increase in lipid peroxidation and for following the effectiveness of the treatment of said pathologies.

Lipid peroxidation is a normal process dealing with the oxidation of polyunsaturated fatty acids (PUFA) which occurs with either fatty acyl or dioxygen radicals [1-3]. Fatty acyl radicals may be issued from PUFA by specific lipoxygenase-catalyzed hydrogen abstraction followed by molecular oxygen binding, ultimately leading to esterified and non-esterified fatty acid hydroperoxides, depending on the status of the PUFA before peroxidation. Such compounds may also arise from autoxidation with less carbon specificity. Those hydroperoxides are normally reduced to the corresponding alcohols by glutathione peroxidase enzymes [4] but they may transiently accumulate if the glutathione peroxidase activity is lowered as described in aging and diabetes [5].
Alternatively, increased amounts of fatty acid hydroperoxides may be produced in response to oxidative stress, whatever its origin, which has been reported in several degenerative diseases including neurodegenerative ones [6-9]. The formation of lipid peroxidation products has been disclosed in alcohol-induced inflammation in liver, in ozone-induced pulmonary inflammation, in pathologies associated with aging and many age-related or chronic diseases including atherosclerosis, diabetes mellitus, rheumatoid arthritis and neurodegenerative diseases such as Alzheimer's and Parkinson diseases. Resulting from elevated fatty acid hydroperoxides at a given time, more breakdown products such as aldehydes can be formed, eventually leading to covalently modified proteins [10-15], nucleic acids [16-18] and lipids [19] through a variety of chemical reactions involving primary amine and thiol groups [20-23] of these biomolecules.
Several biomarkers have already been proposed to evaluate lipid peroxidation but they suffer from either lack of specificity or to be hardly representative of oxidative stress in the whole. As a matter of fact, urinary malondialdehyde is a global marker of PUFA [24-26], including prostaglandin formation as it may derive from the cleavage of the cyclopentane moiety of prostaglandin endoperoxides. Isoprostanes, like 8-epi-PGF₂α, are specific of the oxygen radical-induced oxidation of arachidonic acid [27-30], but they does not reflect the oxidation of other major PUFA as linoleic and docosahexaenoic acids, although in the latter case neuroprostanes have been described [31,32]. On the other hand, a highly reactive aldehyde, 4-hydroxy-2E-nonenal (4-HNE), is produced from the most abundant n-6 PUFA-derived hydroperoxides [33-36], 15-hydroperoxy-arachidonic acid (15-HpETE) and 13-hydroperoxy-linoleic acid (13-HpODE). Also, 4-hydroxy-2*E*-hexenal (4-HHE) has been described as a major degradation product of n-3 PUFA-derived hydroperoxides [37].

Therefore, lipid peroxidation markers with greater sensitivity and selectivity would be useful for, among other things, the diagnosis of pathologies involving an increase in lipid peroxidation and for following the efficiency of the treatment of these pathologies.

In the present invention, we describe the occurrence of the stable carboxylic acid derivatives in human urine, namely 4-hydroxy-2*E*-nonenoic (4-HNA) and 4-hydroxy-2*E*-hexenoic (4-HHA) acids. 4-hydroxy-2*E*-nonenoic acid (4-HNA) and 4-hydroxy-2*E*-hexenoic (4-HHA) acids respectively correspond to the following formulas:

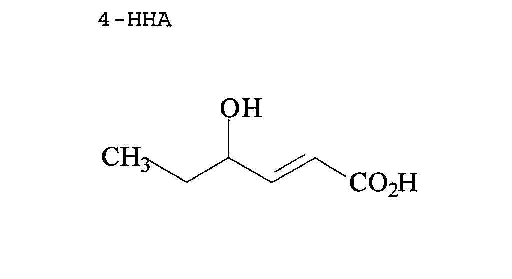

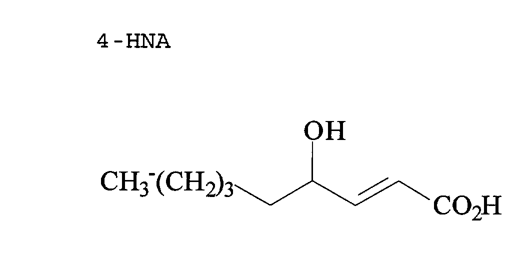

We also describe the formation of an aldehyde, identified as 4-hydroxy-2*E*,6*Z*-dodecadienal (4-HDDE), from the 12-lipoxygenase product of arachidonic acid 12-HpETE, and its carboxylic acid derivative, 4-hydroxy-2E,6Z-dodecadienoic acid (4-HDDA) which corresponds to the following formula :

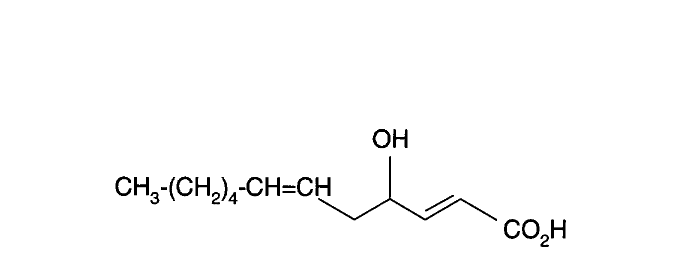

Polyunsaturated fatty acids of the n-6 and n-3 series are expected to be peroxidized on their n-6 or n-3 carbon, respectively, to provide hydroperoxides which are normally reduced by glutathione peroxidases. The two main breakdown products of those hydroperoxides are 4-hydroxy-2*E*-nonenal (4-HNE) and -hexenal (4-HHE), respectively. Their carboxylic acid derivatives, 4-hydroxy-2*E*-nonenoic (4-HNA) and -hexenoic (4-HHA) acids, were synthesized and characterized by nuclear magnetic resonance (NMR) and gas chromatography - mass spectrometry (GC-MS). They were detected in human urine by GC-MS and measured at pg levels by negative ion chemical ionization (NICI) GC-MS using their corresponding internal standards labeled with deuterium at their methyl terminus (CD₃). An easy detection could be done with 2 to 3 ml of urine, and 2.31 ± 0.39 and 1.21 ± 0.37 ng/µmol creatinine were respectively found in healthy human volunteers (n = 15).
On the other hand, a homolog of these two markers 4-hydroxy-2*E*,6*Z*-dodecadienoic acid (4-HDDA), has also been synthesized, characterized and measured in urine, as the carboxylic acid derivative of 4-hydroxy-2*E*,6*Z*-dodecadienal (4-HDDE) issued from the 12-lipoxygenase product of arachidonic acid 12-HpETE. Also measured by NICI-GC-MS with the corresponding terminal CD₃ analog as an internal standard, 4.16 ± 1.02 ng/µmol creatinine were found in healthy human volunteers (n = 15). Finally, some significant differences could be observed in diabetic patients when compared with values obtained in healthy volunteers. In conclusion, the present invention reports the characterization of new specific markers of lipid peroxidation in human urine, with 4-HNA representing all n-6 fatty acids (mainly arachidonic and linoleic acids), 4-HHA deriving from n-3 fatty acids (mainly docosahexaenoic acid) and 4-HDDA as an index of the 12-lipoxygenation of arachidonic acid. Differential increase in those markers may be observed in pathophysiologic states.
As a consequence, the present invention concerns 4-hydroxy-2E-alkenoic acid according to formula (I) below 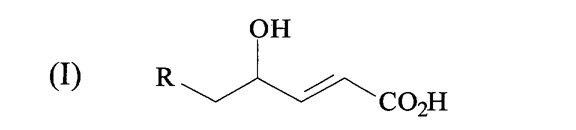 in which R is chosen in the group consisting of CH₃-, and CH₃-(CH₂)₄-CH=CH-.

The present invention also concerns the use of a 4-hydroxy-2E-alkenoic acid according to formula (I) below 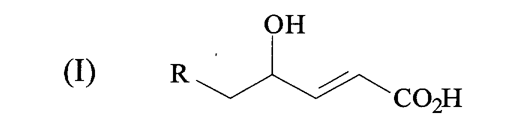 in which R is chosen in the group consisting of CH₃-, CH₃-(CH₂)- and CH₃-(CH₂)₄-CH=CH- as lipid peroxidation marker. More particularly, 4-HNA in which R is CH₃- (CH₂) ₃- is used as marker of n-6 fatty acid lipid peroxidation while 4-HHA in which R is CH₃-is used as a marker of n-3 fatty acid lipid peroxidation. Moreover, 4-HDDA in which R is CH₃-(CH₂)₄-CH=C_{H}- is used as marker of the 12-lipoxygenation of arachidonic acid.
Thus, the present invention concerns a method for analysing lipid peroxidation comprising the detection and/ or the quantification of at least a 4-hydroxy-2*E*-alkenoic acid according to formula (I) already disclosed in which R is chosen in the group consisting of CH₃-, CH₃- (CH₂) ₃- and CH₃- (CH₂)-CH=CH-.

The present invention also concerns a method for determining the presence of a 4-hydroxy-2*E*-alkenoic acid according to formula (I) already disclosed in which R is chosen in the group consisting of CH₃-, CH₃- (CH₂) ₃- and CH₃- (CH₂)₄-CH=CH- in solution and eventually detecting the level of said 4-hydroxy-2*E*-alkenoic acid.
According to the present invention, the method for determining the presence and, eventually, the level of a 4-hydroxy-2*E*-alkenoic acid in solution comprises the following steps:
i) adding an internal standard to said solution,
ii) extracting and purifying 4-hydroxy-2*E*-alkenoic acid and the internal standard present in said solution,
iii) submitting said 4-hydroxy-2*E*-alkenoic acid and said internal standard extracted and purified at step (ii) to a pentafluorobenzylester derivatization procedure,
iv) converting the hydroxyl group of the pentafluorobenzylester derivatives obtained at step (iii) into trimethylsilyl ether,
v) eventually analysing the pentafluorobenzylester trimethylsilylether derivatives (PFB-TMS derivatives) obtained at step (iv) by gas chromatography mass spectrometry (GC-MS),
vi) quantifying said 4-hydroxy-2*E*-alkenoic acid according to the amount of the internal standard present in said solution.

The internal standard advantageously used at step (i) in the present method to determine the level of the 4-hydroxy-2*E*-alkenoic acids is a labelled 4-hydroxy-2*E*-alkenoic acid. Preferably, such acid is labelled at its methyl terminus using an isotope and, more particularly, deuterium. The deuterium-labelled 4-hydroxy-2*E*-alkenoic acid used is preferably one selected in the group consisting of 4-HHA-d₃, 4-HNA-d₃ and 4-HDDA-d₃. A known quantity of internal standard is used to let quantification of the 4-hydroxy-2*E*-alkenoic acid eventually present in the solution.

At step (ii), any method known by the one skilled in the art can be used to extract and purify 4-hydroxy-2*E*-alkenoic acids and the internal standard. It can be cited, for example, extraction using anion exchange colum....

The pentafluorobenzylester derivatization procedure implemented at step (iii) is a well-known procedure for the one skilled in the art. This procedure can be performed using α-bromo-2,3,4,5,6-pentafluorotoluene as previously disclosed in Bordet et al. (45).

The step (iv) in the method object of the present invention is advantageously performed by treating the pentafluorobenzylester derivatives with *N*,*O*-Bis-(trimethylsilyl)-trifluoroacetamid.

At step (v), the analysis of the pentafluorobenzylester trimethylsilylether derivatives is advantageously performed using the negative ion chemical ionization mode (NICI). The negative ionization mass spectra of each 4-hydroxy-2E-alkenoic acid provides one fragment which corresponds to the loss of the pentafluorobenzyl ester group (ion [M-181]⁻). Such ions are observed at m/z 243, 201 and 283 respectively for 4-HNA, 4-HHA and 4-HDDA.

Moreover, the determination of the level of the 4-hydroxy-2*E*-alkenoic acids contained in a solution as performed at step (vi) is also performed using the negative ion chemical ionization mode (NICI) and quantitation was carried out by selected ion monitoring using the [M-181]- ion, m/z 243 for 4-HNA, m/z 201 for 4-HHA, m/z 283 for 4-HDDA, m/z 246 for 4-HNA-d₃, m/z 204 for 4-HHA-d₃, and m/z 286 for 4-HDDA-d₃.

The present invention also concerns a method for diagnosing in a subject a pathology involving an increase in lipid peroxidation using a biological fluid from said subject.
"Pathology involving an increase in lipid peroxidation" refers to any pathology known in which an increase in lipid peroxidation has been reported. Such pathologies are, for example, alcohol-induced inflammation in liver, ozone-induced pulmonary inflammation, age-related or chronic diseases including atherosclerosis, diabetes mellitus, rheumatoid arthritis and neurodegenerative diseases such as Alzheimer's and Parkinson diseases. The present invention also covers the pathologies in which an increase in lipid peroxidation exists but has not yet been described.
"Biological fluid" refers to a fluid extracted from a subject before the implementation of a method according to the present invention. Such biological fluid can be chosen among, for example, the blood, serum, plasma, urine, saliva or sweat of said subject. Advantageously, in the present invention, the biological fluid used is urine.
"Subject" refers, in the present invention, to an animal, such as a mammal including a human, male or female, healthy or suspected of suffering or suffering from a pathology involving an increase in lipid peroxidation or from any other disease.
The method for diagnosing a pathology involving an increase in lipid peroxidation, object of the present invention consists in:
a) measuring the level of at least one 4-hydroxy-2E-alkenoic acid chosen in the group consisting in 4-HHA, 4-HNA and 4-HDDA in a biological fluid from a subject according to the methods for determining their level in solution already described,
b) comparing the level measured with a reference level representing the absence of pathology.

The reference level representing the absence of pathology can be easily obtainable for the one skilled in the art by testing the biological fluids from several healthy subject and determining in these biological fluids the level of 4-HHA, of 4-HNA and/or of 4-HDDA according to the methods already described. If the method for diagnosing uses a particular biological fluid, the one skilled in the art will use the same biological fluid from healthy subjects to determine the different reference levels.
Advantageously, the method for diagnosing a pathology involving an increase in lipid peroxidation is done by using urine as biological fluid. The reference levels in urine are close to 2, 1, and 4 ng/µmol creatinine of 4-HNA, 4-HHA and 4-HDDA, respectively. Nevertheless, it will be very easy for the one skilled in the art to check these reference levels according to the experimental conditions used to determine the 4-hydroxy-2*E*-alkenoic acid levels if they are different as the ones disclosed in the examples below.
Moreover, before implementing the method for determining the 4-hydroxy-2*E*-alkenoic acid levels in the biological fluid, this fluid can undergo treatments such as, for example, dilution, concentration, alcalinization, ....
In the method of diagnosing a pathology involving an increase in lipid peroxidation, only one of the specific markers of the lipid peroxidation chosen among 4-HHA, 4-HNA and 4-HDDA can be measured. Alternatively, the diagnosis can also be done by measuring, in the biological fluid, two or all of these markers and by comparing ratio of different markers with a reference ratio. The comparison of ratios is an index of preferential peroxidation over the other ones. The example below illustrates this aspect of the present invention. As, in this example, the ratio between 4-HHA and 4-HNA studied in diabetic subjects is an index of preferential peroxidation of n-3 fatty acids over n-6 ones.

In another aspect of the present invention, the method for determining the level of a 4-hydroxy-2*E*-alkenoic acid chosen in the group consisting in 4-HHA, 4-HNA and 4-HDDA in solution can be used to follow the effectiveness of the treatment of pathology involving an increase in lipid peroxidation. The method for following said effectiveness consists in:
a') measuring the level of at least one 4-hydroxy-2*E*-alkenoic acid chosen in the group consisting in 4-HHA, 4-HNA and 4-HDDA in a biological fluid from a subject according to the methods for determining their level in solution already described,
b') comparing the level measured with a reference level obtained without any treatment.

The present method for following the efficiency of a treatment applies to a particular subject suffering from a pathology involving an increase in lipid peroxidation and treated for this pathology. The reference level obtained without any treatment can be easily obtainable for the one skilled in the art by testing a chosen biological fluid of said subject and determining in it the level of 4-HHA, of 4-HNA and/or of 4-HDDA according to the methods already described, before any treatment. The following measurements will be done after starting the treatment and on the same kind of biological fluid, the one skilled in the art will appreciate the frequency and the time to realize such measurements.

Advantageously, the method for following the efficiency of a treatment is done by using urine as biological fluid.
Moreover, before implementing the method for determining the 4-hydroxy-2*E*-alkenoic acid levels in the biological fluid, this fluid can undergo treatments such as, for example, dilution, concentration, alcalinization, ....
In the method for following the efficiency of a treatment applies to a particular subject suffering from a pathology involving an increase in lipid peroxidation and treated for this pathology, only one of the specific markers of the lipid peroxidation chosen among 4-HHA, 4-HNA and 4-HDDA can be measured. Alternatively, the following can also be done by measuring, in the biological fluid, two or all of these markers and by comparing ratio of different markers with a reference ratio.

Then, the present invention concerns kits for the implementation of any method previously disclosed comprising means for measuring the level of a 4-hydroxy-2*E*-alkenoic acid and at least a labelled 4-hydroxy-2*E*-alkenoic acid. Preferably, such acid is labelled at its methyl terminus using an isotope and, more particularly, deuterium. The deuterium-labelled 4-hydroxy-2*E*-alkenoic acid used is preferably one selected in the group consisting of 4-HHA-d₃, 4-HNA-d₃ and 4-HDDA-d₃.

Other advantages and characteristics of the invention will become apparent by reading the following examples concerning the syntheses of 4-HDDE and 4-hydroxy-2E-alkenoic acids and the characterization of said acid metabolites in human urine and in which:

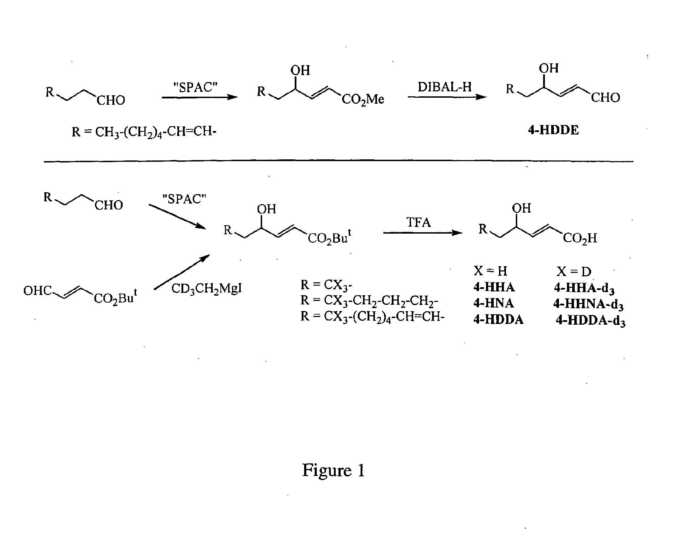

Figure 1 is a scheme of the syntheses of 4-HDDE and 4-hydroxy-2E-alkenoic acids.

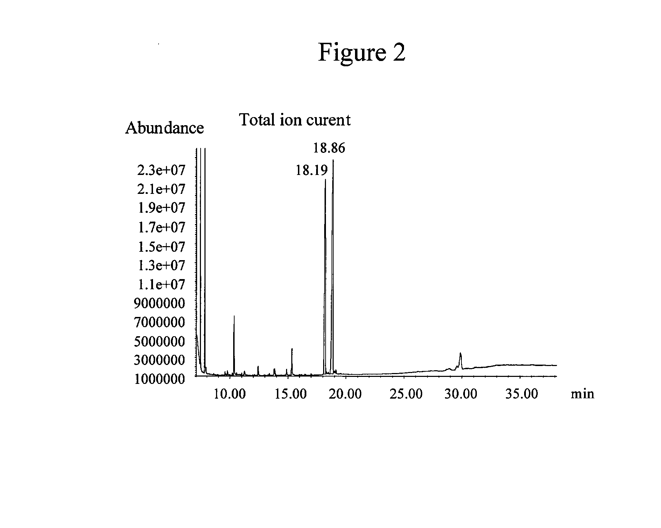

Figure 2 represents the total ion current of chemically synthesized 4-hydroxy-(2E,6Z)-dodecadienal (4-HDDE) derivatized as *O*-pentafluorobenzyl oxime, trimethylsilyl ether. The GC separation was done with a HP-1MS fused-silica capillary column (30 m x 0.25 mm i.d., 0.25 µm film thickness) (Hewlett Packard) which was held at 57°C. The following oven temperature program was used: 2 min at 57 °C, then increased to 180 °C at a rate of 20 °C/min, followed by an increase to 280 °C at a rate of 4 °C/min. The interface, injector and ion source were kept at 280, 280 and 130°C, respectively. Helium was used as a carrier gas The negative chemical ionization (NICI) was performed with methane as reactant gas. The electron multiplier voltage was usually set at 1400 V.

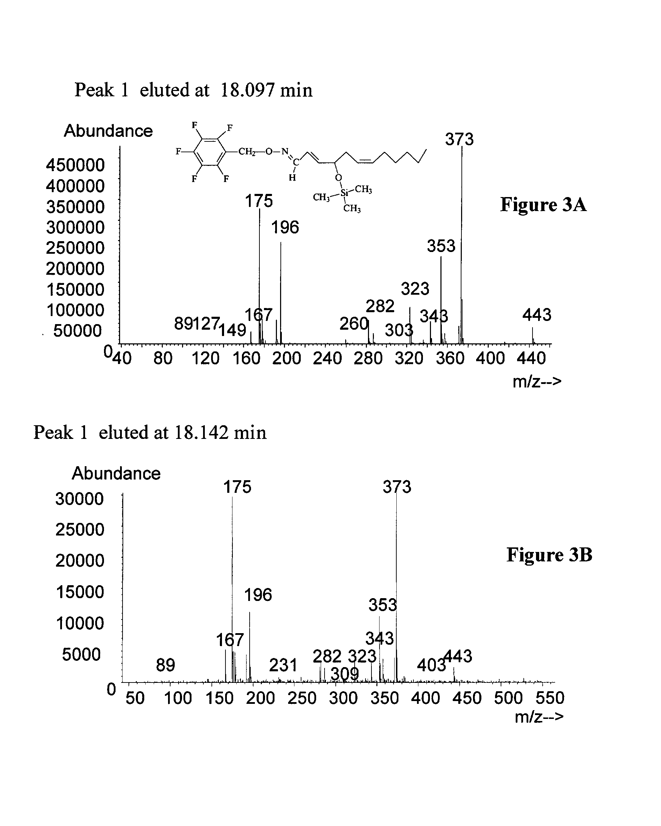

Figure 3 represents the NICI mass spectrum of 4-HDDE derivatized as *O*-pentafluorobenzyl oxime, trimethylsilyl ether. Analytical conditions were the same as described in Figure 2. The mass spectrum was acquired from 50 to 800 Da using the NICI mode. 3A represents the NICI mass spectrum of the first isomer of standard 4-HDDE (eluted at 18.09 min) (see figure 2) and 3B the mass spectrum of the same isomer of 4-HDDE originating from plasma (eluted at 18.14 min).

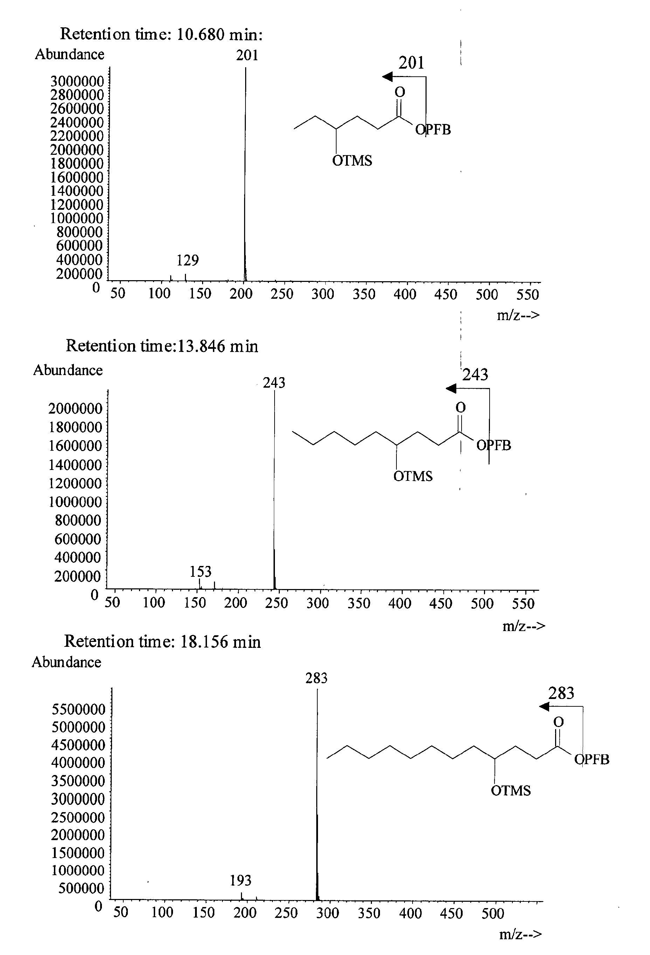

Figure 4 represents the NICI mass spectrum of chemically synthesized 4-HHA, 4-HNA and 4-HDDA derivatized as pentafluorobenzyl ester, trimethylsilyl ether. Analytical conditions were the same as described in Figure 3.

### I. MATERIALS AND METHODS.

All chemicals and reagents were analytical grade and purchased from Sigma-Fluka-Aldrich Chemical Co, (St Quentin Fallavier, France). Analytical grade organic solvents, silica gel 60 (40-63 µm) for column chromatography and silica thin-layer chromatography (TLC) plates were purchased from Merck (Nogent/Marne, France). [³H]-arachidonic acid was from N.E.N. Dupont de Nemours (Les Ulis, France). 7,7,7-Trideuteroheptanal and 10,10,10-trideudetero-4Z-decenal were elaborated by classical reactions, respectively in a four step synthesis from 6-bromo-1-hexanol and trideuteromethylmagnesium iodide (total yield 49 %) and in a six step synthesis from 4-pentyn-1-ol, 1.4-dibromobutane and trideuteromethylmagnesium iodide (total yield 18 %). Tertiobutyl 4-oxo-2*E*-butenoate was prepared by ozonolysis of tertiobutyl sorbate [38] (yield 73 %). Nuclear magnetic resonance spectra were recorded in CDCl₃ with a Bruker AC 200 (200/50 MHz) spectrometer. Chemical shifts are given in ppm and are referenced to CHCl₃ resonances (7.26 and 77.0 ppm). Splitting pattern abbreviations are s, singlet ; d, doublet ; dd, doublet of doublet ; ddd, doublet of doublet of doublet ; t, triplet ; sept, septuplet, m, multiplet, br, broad.

### 1.1. Synthesis of 4-hydroxy-2E,6Z-dodecadienal (4-HDDE).

Methyl 4-hydroxy-2E,6Z-dodecadienoate was obtained by a "SPAC" (Sulfoxide Piperidine and Carbonyl) reaction between methyl 4-chlorophenylsulfinylacetate and 4*Z*-decenal, following the method of Tanikaga et al. [39]. The crude product was purified by flash chromatography on silica gel with dichloromethane/ether (19:1, v/v) as eluent to afford methyl 4-hydroxydodeca-2*E*,6Z-dienoate in a 71 % yield.

### 1.2. Preparation of 4-HDDE.

To a stirred solution of methyl 4-hydroxydodeca-(2E,6Z)-dienoate (202 mg, 0.89 mmol) in anhydrous dichloromethane (10 ml) at -90°C under nitrogen was added dropwise a solution of 1.8 ml of 1M diisobutylaluminium hydride (DIBAL-H) in anhydrous dichloromethane diluted with 10 ml of anhydrous dichloromethane. The temperature was allowed to rise to - 75/-70 °C and the mixture was stirred for 1 h. A 1M HCl aqueous solution (10 ml) was then added at -70°C and the mixture allowed to warm to room temperature. The organic layer was separated and dried over anhydrous Na₂SO₄. The solvent was evaporated under reduced pressure and the residue was purified by flash chromatography on silica gel with pentane/ether (1:1, v/v) as eluent to afford 4-HDDE (77 mg, yield 44 %).
*4-HDDE* : ¹H-NMR (CDCl₃) δ = 9.59 (d, J = 7.8 Hz, H-1); 6.85 (dd, J = 15.7, 4.3 Hz, H-3); 6.35 (ddd, J = 15.7, 7.8, 1.4 Hz, H-2); 5.73-5.60 (m, H-6); 5.45-5.31 (m, H-7); 4.60-4.40 (m, H-4); 2.43 (t, J = 7.0 Hz, 2H-5); 2.08-2.00 (m, 2H-8); 1.45-1.20 (m, 2H-9, 2H-10, 2H-11) ; 0.89 (t, J = 7 Hz, 3H-12) . ¹³C-NMR (CDCl₃) δ = 193.61 (C-1); 158.40 (C-3); 135.10 (C-7); 130.92 (C-6); 122.90 (C-2); 70.54 (C-4); 34.57 (C-5); 31.50 (C-10); 29.21 (C-9); 27.44 (C-8); 22.54 (C-11); 14.04 (C-12).

### 1.3. Syntheses of 4-hydroxy-2E-alkenoic acids (4-HHA, 4-HHA-d₃, 4-HNA, 4-HNA-d₃, 4-HDDA and 4-HDDA-d₃ ) .

The *tert*-butyl esters of 4-HHA, 4-HNA, 4-HNA-d₃ , 4-HDDA and 4-HDDA-d₃ were obtained by a slightly modified "SPAC" (Sulfoxide Piperidine and Carbonyl) reaction according to the following procedure: to a solution of *tert*-butyl 4-chlorophenylsulfinylacetate (1.8 g, 6.55 mmol), piperidine · (0.8 ml, 8.0 mmol) and 1.4-diazabicyclo[2.2.2]octane (Dabco™) (0.75 g, 6.7 mmol)) in acetonitrile (16 ml) and water (0.4 ml), under nitrogen, was added dropwise a solution of aldehyde (8.53 mmol) in acetonitrile (2 ml). The mixture was stirred overnight at room temperature. The solvent was evaporated under reduced pressure. The crude *tert*-butyl esters were purified by flash chromatography on silica gel eluting with dichloromethane/methanol (97:3, v/v) for 4-HHA *tert*-butyl ester, dichloromethane /ether (90:10, v/v) for 4-HNA *tert*-butyl ester and 4-HNA-d₃ tert-butyl ester or dichloromethane/ether (98:2, v/v) for 4-HDDA *tert*-butyl ester and 4-HDDA-d₃ *tert*-butyl ester. The yields were in the range 80-90 %.
The *tert*-butyl ester of 4-HHA-d₃ was prepared as follows: to a stirred solution tert-butyl 4-oxo-2E-butenoate (2.5 g, 16 mmol) in anhydrous ether (5 ml) at -78°C under nitrogen was added, over a period of 1 h, an ethereal solution of 2.2.2-trideuteroethylmagnesium iodide, prepared from magnesium (403 mg, 16.58 mmol) and 2,2,2-trideuteroethyl iodide (2.8 g, 17.61 mmol) in anhydrous ether (30 ml) (30) . After stirring at -78°C for 2 h, the resulting mixture was quenched at the same temperature with a 0.5 M HCl aqueous solution (60 ml). After extraction with ether (2x50 ml), the organic phase was washed with brine (2x50 ml), dried over anhydrous Na₂SO₄ and evaporated under reduced pressure. The residue was purified by flash chromatography on silica gel (160 g) eluting with dichloromethane/ether (85:15, v/v) to afford pure 4-HHA-d₃ *tert*-butyl ester (1.25 g, yield 40 %).
The 4-hydroxy-2*E*-alkenoic acids were obtained by acidic cleavage of the corresponding esters in the following way: to a stirred solution of tert-*butyl* 4-hydroxy-2E-alkenoate (5.57 mmol) in dichloromethane (1.7 ml) cooled in a water ice bath was added dropwise TFA (6.5 ml). The cooling bath was removed and the solution was stirred for 20 mn at room temperature. The solvent was evaporated under reduced pressure. The residue was purified by flash chromatography on silica gel eluting with dichloromethane/ether (60:40, v/v) for 4-HHA, 4-HHA-d₃, 4-HNA and 4-HNA-d₃ or dichloromethane/ether (80:20, v/v) for 4-HDDA and 4-HDDA-d₃. The yields were in the range 85-90 %.
*4-HHA* : ¹H-NMR (CDCl₃) δ = 7.06 (dd, J = 15.7, 4.7 Hz, H-3); 6.07 (dd, J = 15.7, 1.6 Hz, H-2); 5.50 (br s, 2 OH) ; 4.30 (m, H-4); 1.80-1.50 (m, 2H-5); 0.99 (t, J = 7.4 Hz, 3H-6) . ¹³C-NMR (CD₃OD) δ = 170.31 (C-1) ; 152.70 (C-3) ; 121.38 (C-2) ; 73.14 (C-4) ; 30.68 (C-5) ; 10.27 (C-6).
*4-HHA-d*_{*3*} : ¹H-NMR (CDCl₃) δ = 7.06 (dd, J = 15.7, 4.7 Hz, H-3) ; 6.07 (dd, J = 15.7, 1.7 Hz, H-2) ; 5.94 (br s, 2 OH); 4.30 (m, H-4); 1.80-1.50 (m, 2H-5). ¹³C-NMR (CD₃OD) δ = 170.31 (C-1) ; 152.72 (C-3) ; 121.38 (C-2) ; 73.12 (C-4) ; 30.45 (C-5) ; 9.43 (sept, ¹J_{CD} = 19.2 Hz, C-6).
*4-HNA* : ¹H-NMR (CDCl₃) δ = 7.06 (dd, J = 15.7, 4.8 Hz, H-3); 6.75 (br s, 2 OH); 6.06 (dd, J = 15.7, 1.6 Hz, H-2); 4.35 (m, H-4); 1.70-1.50 (m, 2H-5); 1.50-1.20 (m, 2H-6, 2H-7, 2H-8); 0.90 (t, J = 6.4 Hz). ¹³C-NM_{R} (CDCl₃) ) δ = 171.46 (C-1) ; 152.64 (C-3) ; 119.59 (C-2) ; 71.20 (C-4) ; 36.36 (C-5) ; 31.65 (C-8) ; 24.89 (C-6 or C-7) ; 22.54 (C-7 or C-6) ; 14.00 (C-9).
*4-HNA-d*_{*3*} *:* ¹H-NMR (CDCl₃) δ = 7.07 (dd, J = 15.7, 4.8 Hz, H-3); 6.78 (br s, 2 OH); 6.06 (dd, J = 15.7, 1.6 Hz, H-2); 4.36 (m, H-4); 1.70-1.50 (m, 2H-5); 1.50-1.20 (m, 2H-6, 2H-7, 2H-8). ¹³C-NMR (CDCl₃) δ = 171.51 (C-1) ; 152.66 (C-3) ; 122.52 (C-2) ; 71.23 (C-4) ; 36.37 (C-5) ; 33.38 (C-8) ; 24.90 (C-6 or C-7) ; 22.26 (C-7 or C-6) ; 13.09 (sept, ¹J_{CD} = 19.1 Hz, C-9).
*4-HDDA* : ¹H-NMR (CDCl₃) δ = 7.08 (dd, J = 15.6, 4.3 Hz, H-3); 6.80-6.20 (br s, 2 OH); 6.10 (dd, J = 15.6, 1.7 Hz, H-2); 5.73-5.59 (m, H-6); 5.45-5.31 (m, H-7); 4.44-4.35 (m, H-4); 2.44-2.30 (m, 2H-5); 2.15-1.90 (m, 2H-8); 1.50-1.15 (m, 2H-9, 2H-10, 2H-11); 0.89 (t, J = 6.6 Hz, 3H-12). ¹³C-NMR (CDCl₃) δ = 171.46 (C-1); 151.92 (C-3); 134.89 (C-7); 123.07 (C-6); 119.83 (C-2); 70.56 (C-4); 34.50 (C-5); 31.50 (C-10); 29.22 (C-9); 27.42 (C-8); 22.56 (C-11); 14.04 (C-12).
*4-HDDA-d*_{*3*} : ¹H-NMR (CDCl₃) δ = 7.08 (dd, J = 15.6, 4.3 Hz, H-3); 6.70-6.50 (br s, 2 OH); 6.09 (dd, J = 15.6, 1.7 Hz, H-2); 5.80-5.55 (m, H-6); 5.55-5.25 (m, H-7); 4.50-4.30 (m, H-4); 2.58-2.25 (m, 2H-5); 2.15-1.95 (m, 2H-8); 1.50-1.10 (m, 2H-9, 2H-10, 2H-11). ¹³C-NMR (CDCl₃) δ = 171.60 (C-1) ; 151.96 (C-3); 134.95 (C-7); 123.02 (C-6); 119.81 (C-2); 70.57 (C-4); 34.51 (C-5); 31.43 (C-10); 29.24 (C-9); 27.43 (C-8); 22.29 (C-11); 13.14 (sept, ¹J_{CD} = 19.1 Hz, C-12) .

### 1.4. Preparation of biological samples.

### a. Platelets.

Blood platelets were isolated from human volunteers (local blood bank) according to Lagarde et al. [40]. Briefly, blood taken onto anticoagulant (ACD) was centrifuged at 150 g. The supernatant platelet-rich plasma (PRP) was acidified to pH 6.4, centrifuged at 900 g for 10 min, and the pellets were resuspended into Tyrode HEPES buffer pH 7.35.

### b. Urinary samples.

2.5 ml urine were diluted with 2.5 ml of distilled water and alcalinized to pH 7.5 with 100 µl of 1 M NaOH for 30 min under gentle shaking. Samples were then extracted using a 6 ml anion exchange Sep-Pak cartridge (Interchim, Montluçon, France) which has been washed with 6 ml of methanol and 6 ml distilled water before the urine deposit. Urinary acids were eluted with 6 ml 0.2 M citric phosphate buffer pH 2.4 containing 10% methanol. The aqueous phase was immediately extracted twice with 5 ml chloroform/ethanol (2:1 by volume). Samples were then transferred into conical vials for their further derivatization.

### 1.5. 12-HpETE and aldehyde breakdown.

Platelet suspension was preincubated under gentle stirring with 200 µM diamide, to lower reduced glutathione and then slow down glutathione peroxidase, and with 200 µM acetylsalicylic acid as a cyclooxygenase inhibitor, for 5 min at 37°C. Platelets were then incubated with 300 µM arachidonic acid labeled with 37 kBq of [5,6,8,9,11,12,14,15-³H]-AA (7.77 TBq/mmol) for 5 min in the presence of oxygen. Platelet suspension was then acidified to pH 3 with 3 M HCl and extracted three times with 3 volumes diethyl ether. The organic phase was evaporated to dryness under vacuum. The 12-HpETE formation was assessed from an aliquot of the lipid extract and TLC using the solvent mixture hexane/diethyl ether/acetic acid (60:40:1, v/v). Labeled compounds were detected by a Berthold TLC linear analyser. The dry lipid extract, before TLC, was treated for 22 hours at room temperature with 5 ml of 0.1 M HCl containing 0.5 M ascorbate and 0.02 M FeSO₄ according to the procedure previously used by Salter et al. (35) to synthesize 4-hydroxynonenal from 15-HpETE. The incubate was extracted with 45 ml of the mixture chloroform/ethanol (2:1, v/v). The organic phase was removed, dried under vacuum and the residue was purified by TLC using the solvent mixture pentane/diethylether (50:50, v/v) as eluent. 30 µg of standard 4-HDDE, used as a reference, was spotted on the same plate, and the radioactive band corresponding to standard was scraped off and extracted three times with chloroform/ethanol (2:1, v/v).

### 1.6. Aldehyde derivatization and purification.

The dry purified aldehyde or crude plasma was treated with 200 µl of *O*-2,3,4,5,6-pentafluorobenzyl hydroxylamine hydrochloride (PFBHA) (50 mM in 0.1 M PIPES buffer pH 6.5) for 30 min at room temperature, according to the procedure described by van Kuij et al. (37) . After acidification with 100 µl of 98 % H₂SO₄, pentafluorobenzyloxime derivatives were treated with 500 µl methanol and 2 ml hexane, and then purified by TLC using pentane/diethylether (70:30, v/v) as eluent. The radioactive band, corresponding to standard pentafluorobenzyl oxime 4-HDDE, was scraped off and the derivative was extracted three times with chloroform/ethanol (2:1, v/v). The solvent was removed under nitrogen and the hydroxyl group was converted into trimethylsilylether after an overnight treatment with *N,O*-Bis(trimethylsilyl)trifluoroacetamide (BSTFA) at room temperature. The pentafluorobenzyl oxime, trimethylsilylether-4-HDDE derivative (*O*-PFB-TMS-4-HDDE) was then analyzed by gas chromatography - mass spectrometry (GC-MS).

### 1.7. GC-MS derivatization of 4-hydroxy-acids.

The organic solvent was removed under nitrogen and the sample was dissolved into 60 µl of acetonitrile, after which 20 µl of 350 g/l α-bromo-2,3,4,5,6-pentafluorotoluene in acetonitrile and 20 µl of N-ethyldiisopropylamine were added, and the mixture was heated at 40°C for 30 min. The pentaf luorobenzyl (PFB) ester derivatives were then treated with 100 µl *N*,*O*-Bis-(trimethylsilyl)-trifluoroacetamid (BSTFA) overnight at room temperature to convert their hydroxyl groups into trimethylsilyl (TMS) ether. One microliter aliquot of pentafluorobenzylester trimethylsilylether derivatives was analyzed by GC-MS in the splitless mode.

### II. RESULTS.

### II.1. Syntheses of 4-HDDE and 4-hydroxy-2E-alkenoic acids (4-HHA, 4-HHA-d₃, 4-HNA, 4-HNA-d₃, 4-HDDA and 4-HDDA-d₃).

All synthesized compounds were obtained as racemic mixtures. 4-HDDE [42] was prepared by reduction of methyl 4-hydroxy-2*E*,6*Z*-dodecadienoate with diisobutylaluminium hydride (DIBAL-H) whereas the 4-hydroxy-2*E*-alkenoic acids were obtained by acidic cleavage of the corresponding tertiobutyl esters with trifluoroacetic acid (TFA). Methyl 4-hydroxy-2*E*,6*Z* dodecadienoate and the tertiobutyl esters of 4-HHA, 4-HNA, 4-HNA-d₃, 4-HDDA and 4-HDDA-d₃ were elaborated by a "SPAC" (Sulfoxide Piperidine and Carbonyl) reaction [39,43-44] between methyl or tertiobutyl 4-chlorophenylsulfinylacetate and the requisite aldehyde. Owing to the difficulty to prepare the volatile 4,4,4-trideuterobutanal on a small scale, the tertiobutyl ester of 4-HHA-d₃ was obtained by reaction of tertiobutyl 4-oxo-2E-butenoate with 2.2.2-trideuteroethylmagnesium iodide. All synthesized compounds gave nuclear magnetic resonance spectra in full agreement with the expected structures.
The syntheses of 4-HDDE and 4-hydroxy-2*E*-alkenoic acids are schematized in figure 1.

### 11.2. Characterization of the new peroxide by-product from the 12-lipoxygenase product 12-HpETE.

12-HpETE was prepared from human blood platelets which are well known to form this 12-lipoxygenase product from arachidonic acid. Once produced, 12-HpETE was treated with ascorbate plus Fe²⁺ in conditions to prepare 4-HNE from 15-HpETE. This yielded an aldehyde homologous to 4-HNE, expected to be 4-hydroxy-2*E*,6*Z*-dodecadienal, that we have called 4-HDDE, corresponding to around 10 % of the initial 12-HpETE. This biological product was compared to the chemically synthesized 4-HDDE on the basis of several criteria including its GC-MS behavior. In particular, the presence of two isomers (syn and anti) when derivatized as O-pentafluorobenzyl oxime, trimethylsilylether (Figure 2) and the same negative ion chemical ionisation (NICI) spectra (Figure 3) assess the detection of such aldehyde in plasma. It appears that the main aldehyde produced from 12-HpETE is identical to chemically synthesized 4-HDDE. This indicates that the physico-chemical degradation of 12-HpETE, when the hydroperoxide may escape from reduction by glutathione peroxidase, allows the formation of substantial quantities of 4-HDDE. 4-HDDE as well 4-HNE could easily be measured in human plasma at quantities arounding 1 and 500 ng/mL, respectively. Interestingly, preliminary data obtained from diabetics, matched with controls for age and sex, show that 4-HDDE was significantly higher (p< 0.05) in diabetic plasma than in controls, while 4-HNE did not change.

### 11.3. Characterization of acid metabolites from 4-HNE, 4-HHE and 4-HDDE in human urine.

As those aldehydes may in principle be oxidized into their carboxylic acid metabolites, 4-HNA, 4-HHA and 4-HDDA, respectively, we prepared such acid metabolites as well as their corresponding CD₃ labeled compounds by chemical synthesis and looked for their possible occurrence in urine. The three chemically synthesized acids have been characterized by NMR (see Materials and Methods) and their pentafluorobenzylester, trimethylsilylether derivatives (PFB-TMS) analyzed by GC-MS (Figure 4). The three acid derivatives were found in human urine and characterized by NICI-GC-MS in comparison with the chemically synthesized standards. In order to be sensitive enough, they were measured by single ion monitoring from NICI-GC-MS in using deuterium labeled (CD₃) internal standards first characterized by NMR and GC-MS as done with the non-labeled standards. The ion detected for each compound was the M-181 one from the PFB-TMS derivative, i.e. 243, 201, and 283 from 4-HNA, 4-HHA, and 4-HDDA, respectively. By increased amounts of standards to one urinary sample, a good linear response could be found till 600 ng added to 5 ml urine :
- y = 0.0043x + 0.303; R² = 0.965 (4-HHA),
- y = 0.0037x + 0.414; R² = 0.951 (4-HNA),
- y = 0.0039x + 0.143; R² = 0.986 (4-HDDA) .
The origin ordinate obtained for each product is a precise evaluation of those urinary metabolites. The values obtained from 15 different samples of urine are close to 2, 1, and 4 ng/µmol creatinine of 4-HNA, 4-HHA, and 4-HDDA, respectively. In urine from diabetics matched with age and sex, the values are much more variable, but interestingly 4-HNA and 4-HDDA, but not 4-HNA, were higher than in the control group.

### III. DISCUSSION.

4-HNE is an aldehyde which has been widely described as a product of lipid peroxidation and a reactive one to covalently bind biomolecules having available primary amine and thiol groups. The present results first report on the occurrence of a homolog of 4-HNE issued from the 12-lipoxygenase product of arachidonic acid, 12-HpETE, namely 4-hydroxy-2*E*,6*Z*-dodecadienal (4-HDDE). As such, 4-HDDE may be considered as more specific for arachidonic acid peroxidation than 4-HNE which can occur from both 15-HpETE and 13-HpODE, the n-6 peroxidation products of arachidonic and linoleic acids, respectively. It is noteworthy that our characterizations and measurements do not discriminate between the R and S stereoisomers, the S ones being expected to derive from lipoxygenase (12- and 15-lipoxygenases) catalysis whereas racemic products are likely to be issued from autoxidation. This means that measuring 4-HNE and 4-HDDE in biological tissues reflects the enzymatic and non-enzymatic peroxidations, but is specific of the oxygen position on the acyl chains. A reliable measurement of 4-HNE and 4-HDDE in human blood plasma was possible and indicates that the former may be much more abundant than the latter. This could be explained considering that 4-HNE derives from two prominent n-6 fatty acids in humans (arachidonic and linoleic acids), originates from the widespread 15-lipoxygenase activity, and could reflect the propensity of polyunsaturated fatty acids to be oxidized closest to their methyl end. In contrast, 12-HpETE, then 4-HDDE, is mainly produced by the 12-lipoxygenation of arachidonic acid which occurs in few places in the body, the main one being blood platelets. 4-HHE could be detected in plasma but its measurement was hardly reliable due to its volatility during extraction procedures. It is assumed that all these aldehydes may occur in biological fluids because their hydroperoxide precursors can escape from reduction by glutathione peroxidases. It is then of interest from preliminary results that 4-HDDE was significantly higher in diabetic plasma than in control ones. Indeed, glutathione peroxidase activities are substantially attenuated in diabetic platelets [5]. In contrast, the absence of variations in 4-HNE could fit with its multiple origin.
It has been reported that 4-HNE is metabolized through several pathways, one being the oxidation into its carboxylic acid metabolite, 4-HNA, although this metabolite is virtually not found in rat urine. We have been able to detect 4-HNA in human urine and easily measure it from 2-3 ml of urine by NICI-GC-MS. From the same urine sample, we also measured the corresponding carboxylic acids from 4-HDDE and 4-HHE, the aldehyde breakdown products from 12-HpETE and n-3 hydroperoxide metabolites, respectively. Surprinsingly, the urinary concentrations of the acid metabolites, (4-HNA, 4-HDDA and 4-HHA) appear to be similar (1 to 4 ng/µmol creatinine), whereas 4-HNE was around 500 fold higher than 4-HDDE in human plasma. The reasons of such discrepancies are not clear, and we may just speculate that the metabolic pathways of 4-HNE and 4-HDDE might be different. As presented and discussed above with 4-HDDE versus 4-HNE, the acid metabolites were also measured in urine from diabetic patients as a preliminary approach of the pathophysiological relevance of those metabolites. Interestingly, and although the values are much more variable in patients, 4-HDDA and 4-HHA were higher in diabetics than in controls while 4-HNA was not different at all, meaning that the trend for the urinary metabolites is quite similar to that for plasma aldehydes. Also interesting is to consider the ratio between 4-HHA and 4-HNA as an index of preferential peroxidation of n-3 fatty acids over n-6 ones. As a consequence of higher amounts of 4-HHA in urine from diabetics, while 4-HNA was not changed, the ratio 4-HHA/4-HNA was higher in diabetics than in controls. The meaning of those preliminary results can not be assessed at that stage of investigation, but such a tendency would not be unlikely if we consider the very high richness of the retina in docosahexaenoic acid, the main endogenous n-3 fatty acid in animal tissue, and retinopathia as a common diabetic complication.
In conclusion, we have characterized new lipid peroxidation products that could be used as specific markers. They are 4-HDDE/4-HDDA, which mainly reflect the peroxidation of arachidonic acid via 12-lipoxygenase, 4-HHA, which is a specific index of n-3 fatty acid peroxidation whereas 4-HNA reflects that of n-6 fatty acids.

### References

1- Esterbauer, H., Schaur, R.J., and Zollner, H. (1991) Free *Radic. Biol. Med.* **11**, 81-128
2- Halliwell, B. (1994)*Nutr. Rev.* **52,** 253-265
3- Porter, N. Caldwell, S.E., and Mills K.A. (1995) Lipids **30,** 277-290
4- Bryant, R. W., and Bailey, J. M. (1980) Biochem. *Biophys. Res. Commun.* **92,** 268-276
5- Muruganandam, A., Drouillard, C., Thibert, R., Cheung, R., Draisey, T., and Mutus, B. (1992) Thromb. Res.67, 385-397
6- Montine, K., Kim, P., Olson, S., Markesbery, W., and Montine, T. (1997) *J. Neuropathol. Exp. Neurol.* **56**, 866-871
7- Neely, M., Sidell, K., Graham, D. and Montine, T. (1999) *J. Neurochem*. **72,** 2323-2333
8- Camandola, S., Poli, G., and Mattson, M. (2000) *Beain Res. Mol. Brain Res.* **85,** 53-60
9- Cao, Q., ong, W., and Halliwell, B. (2001) *Exp. Brain Res.* **137,** 205-213
10- Szweda, L. I., Uchida, K., Tsai, L., and Stadtman, E. R. (1993) *J. Biol. Chem.* **268,** 3342-3347
11- Nadkarni, D. V., and Sayre, L. M. (1995) *Chem. Res. Toxicol.* **8,** 284-291.
12- Malle, E., Ibovnik, A., Leis, H. J., Kostner, G. M., Verhallen, P. F., and Sattler, W. (1995) *Arterioscler. Thromb. Vasc. Biol.* **15,** 377-384
13- Selley, M. L., Bartlett, M. R., Czeti, A**.** L., and Ardlie, N. G. (1998) *Atherosclerosis* **140,** 105-112.
14- Hazen,S., Gaut, J., Crowley, J., Hsu, F. and Heinecke, J. (2000) *Biochem. J.* **352,** 693-699
15- Uchida, K., and Stadtman, E. (2000) *Methods Mol. Biol.* **99,** 25-34
16- Winter, C. K., Segall, H. J., and Haddon, W. F. (1986) *Cancer Res.* **46,** 5682-5686.
17- Wacker, M., Schuler, D., Wanek, P., and Eder, E. (2000) *Chem. Res.* Toxicol. **13,** 1165-1173
18-Morinello, E.J., Ham, A.J., Ranasinghe, A., Sangaiah, R., and Swenberg, J.A. (2001) *Chem. Res. Toxicol*. **14,** 327-334
19- Guichardant, M., Taibi-Tronche, P., Fay, B. L., and Lagarde, M. (1998) *Free Radic. Biol. Med.* **25,** 1049-1056
20- Dogterom, P.,Mulder, G. J., and Nagelkerke, J. F. (1989) *Chem. Biol. Interact.* 71, 291-306.
21- Siems, W. G. Zollner, H., Grune, T., and Esterbauer, H. (1997) *J. Lipid Res.* 38, 612-622.
22- Boon, P. J., Marinho, H. S., Oosting, R., and Mulder, G. J. (1999) *Toxicol. Appl. Pharmacol.* 159, 214-223.
23- Jessup, W., Jurgens, G., Lang, J., Esterbauer, H., and Dean, R. T. (1986) *Biochem.* J. 234, 245-248.
24- Draper,H.H., Polensek, L., Hadley, M. and McGirr, L.G. (1984) *Lipids* **19**: 836-843.
25- Piche, L.A., Draper, H.H., Cole, P.D. (1988) *Lipids* **23**: 370-371.
26- Guichardant, M., Valette Talbi, L., Cavadini C., Crozier G., and Berger, M. (1994) J. *Chromatogr. Biomed. Appl.* **655,** 112-116.
27- Awad, J.A., Morrow, J.D., Takahashi, K. and Roberts, L.J. 2^{nd} (1993) *J. Biol. Chem.* **268:** 4161-4169.
28-Morrow, J.D., Minton, T.A., Mukundan, C.R., Campbell, M.D., Zackert, W.E., Daniel, V.C., Badr, K.F., Blair, I.A. and Roberts L.J. 2nd. (1994) J. Biol. *Chem.* **269:** 4317-4326.
29- Morrow, J.D., Minton, T.A., Badr, K.F. and Roberts L.J. 2nd (1994) 1210: 244-248.
30- Walter, M.F., Blumberg, J.B., Dolnikowski, G.G. and Handelman, G.J. (2000) *Anal. Biochem.* **280,** 73-79.
31- E. Reich, Zackert, W., Brame, C., Chen, Y., Roberts, L., Hachey, D., Montine, T., and Morrow, J. (2000) *Biochemistry* **39,** 2376-2383.
32- E. Reich, Markesbery, W., Y., Roberts, L., Swift, L., Morrow, J., and Montine, T. (2000) *Biochemistry* **39,** 2376-2383.
33- Esterbauer, H. (1982) in *Aldehydic Products of Lipid Peroxidation: Free Radicals Lipid Peroxidation and Cancer* (McBrien, D.C.H. and Slater, T.F. eds) pp 101-128 Academic Press, London
34- Poli, G., Dianzani, M.U., Cheeseman, K.H., Slater, T.F., Lang, J., and Esterbauer, H. (1985) *Biochem. J.* **227**, 629-638.
35- Esterbauer, H., Cheeseman, K.H., Dianzani, M.U., Poli, G., and Slater, T.F. (1982) Biochem. J. 208, 129-140.
36- Comporti, M. (1985) *Lab. Invest.* **53**, 599-623.
37- Van Kuijk, F.J., Holte, LL., Dratz, E.A. (1990) *Biochim. Biophys. Acta* **1043**, 116-118.
38- Veysoglu, T., Mitscher, L.A., and Swayze, J.K. (1980) *Synthesis* 807-811.
39- Tanikaga, R., Nozaki, Y., Tamura, T. and Kaji, A. (1983) *Synthesis* **2**, 134-135.
40- Lagarde, M., Bryon, P. A., Guichardant, M., and Dechavanne, M. (1980) *Thromb. Res.* **17**, 581-588.
41- Lang, J., Celotto, C., and Esterbauer, H. (1985) *Anal. Biochem.* 150, 369-378.
42- Mosset, P., Yadagiri, P., Lumin, S., Capdevilla, J., and Falck, J.R., (1986), *Tetrahedron Letters* **27**, 6035-6038 and 60396040.
43- Burgess, K., Henderson, I. (1990) *Tetrahedron Asymmetry* **1**, 57-60.
44- Allevi, P., Anastasia, M., Ciuffreda, P. and Sanvito, A.M. (1993) *Tetrahedron Asymetry* **4,** 1397-1340.
45- Bordet, J.C., Guichardant, M. and Lagarde M. (1986) *Biochem. Biophys. Res. Commun.* **13,** 403-410.

## Claims

1. A 4-hydroxy-2E-alkenoic acid according to formula (I) below 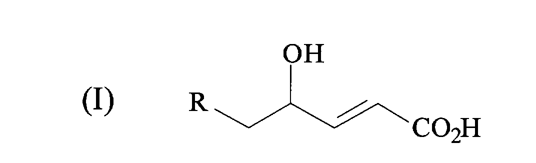 in which R is chosen in the group consisting of CH₃-, and CH₃-(CH₂)₄-CH=CH-.

2. Use of a 4-hydroxy-2*E*-alkenoic acid according to formula (I) below 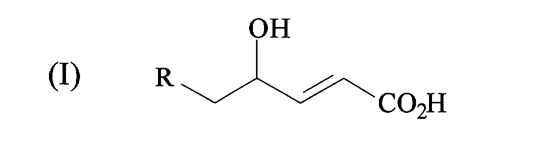 in which R is chosen in the group consisting of CH₃-, CH₃- (CH₂)₃- and CH₃- (CH₂)-CH=CH- as lipid peroxidation marker.

3. Use according to claim 2, wherein the 4-hydroxy-2E-alkenoic acid in which R is CH₃-(CH₂)₃- is a marker of n-6 fatty acid lipid peroxidation.

4. Use according to any of claims 2 or 3, wherein the 4-hydroxy-2E-alkenoic acid in which R is CH₃- is a marker of n-3 fatty acid lipid peroxidation.

5. Use according to any of claims 2 to 4, wherein the 4-hydroxy-2E-alkenoic acid in which R is CH₃- (CH₂) ₄-CH=CH- is a marker of the 12-lipoxygenation of arachidonic acid.

6. Method for analysing lipid peroxidation comprising the detection and/ or the quantification of at least one of 4-hydroxy-2E-alkenoic acid chosen in the group consisting of 4-HHA, 4-HNA and 4-HDDA.

7. Method for determining the presence and, eventually, the level of 4-hydroxy-2E-alkenoic acid chosen in the group consisting of 4-HHA, 4-HNA and 4-HDDA in solution comprising the following steps:
i) adding an internal standard to said solution,
ii) extracting and purifying the 4-hydroxy-2E-alkenoic acid and the internal standard eventually present in said solution,
iii) submitting said 4-hydroxy-2E-alkenoic acid and said internal standard extracted and purified at step (ii) to a pentafluorobenzylester derivatization procedure,
iv) converting the hydroxyl group of the pentafluorobenzylester derivatives obtained at step (iii) into trimethylsilyl ether,
v) analysing the pentafluorobenzylester trimethylsilylether derivatives obtained at step (iv) by gas chromatography mass spectrometry,
vi) eventually, quantifying said 4-hydroxy-2E-alkenoic acid thanks to the internal standard.

8. Method according to claim 7, wherein the internal standard advantageously used at step (i) is a labeled 4-hydroxy-2*E*-alkenoic acid.

9. Method according to any of claims 7 or 8, wherein said internal standard is a 4-hydroxy-2*E*-alkenoic labeled at its methyl terminus with deuterium.

10. Method according to any of claims 7 to 9, wherein the step (iv) is performed by treating the pentafluorobenzylester derivatives with *N,O-*Bis-(trimethylsilyl)-trifluoroacetamid.

11. Method according to any of claims 7 to 10, wherein the analysis of the pentafluorobenzylester trimethylsilylether derivatives at step (v) is performed using the negative ion chemical ionization mode.

12. Method for diagnosing a pathology involving an increase in lipid peroxidation consisting in:
a) measuring the level of at least one 4-hydroxy-2E-alkenoic acid chosen in the group consisting in 4-HHA, 4-HNA and 4-HDDA in a biological fluid from a subject using a method according to any of claims 7 to 11,
b) comparing the level measured with a reference level representing the absence of pathology.

13. Method according to claim 12, wherein the biological fluid used is urine.

14. Method according to any of claims 12 or 13, wherein the reference level are close to 2, 1, and 4 ng/µmol creatinine for 4-HNA, 4-HHA and 4-HDDA, respectively.

15. Method for following the effectiveness of the treatment of pathology involving an increase in lipid peroxidation consisting in:
a') measuring the level of at least one 4-hydroxy-2E-alkenoic acid chosen in the group consisting in 4-HHA, 4-HNA and 4-HDDA in a biological fluid from a subject using a method according to any of claims 7 to 11,
b') comparing the level measured with a reference level obtained without any treatment.

16. Method according to any of claims 12 to 15, wherein said pathology is chosen in the group consisting in alcohol-induced inflammation in liver, ozone-induced pulmonary inflammation, age-related or chronic diseases including atherosclerosis, diabetes mellitus, rheumatoid arthritis and neurodegenerative diseases such as Alzheimer's and Parkinson diseases.

17. Kit for the implementation of a method according to any of claims 12 to 16, comprising means for measuring the level of a 4-hydroxy-2E-alkenoic acid and at least a labelled 4-hydroxy-2*E*-alkenoic acid.
